(19) Europäisches Patentamt / European Patent Office / Office européen des brevets

(11) **EP 4 089 177 A1**

(12) **EUROPEAN PATENT APPLICATION**

(43) Date of publication:
**16.11.2022 Bulletin 2022/46**

(21) Application number: **21174190.5**

(22) Date of filing: **17.05.2021**

(51) International Patent Classification (IPC):
**C12P 7/40** *(2006.01)*       **C12P 7/54** *(2006.01)*
**C12P 7/56** *(2006.01)*       **C12P 7/06** *(2006.01)*
**C12P 7/10** *(2006.01)*

(52) Cooperative Patent Classification (CPC):
**C12P 7/40; C12P 7/065; C12P 7/10; C12P 7/54; C12P 7/56**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.05.2021 PT 2021117228**

(71) Applicant: **Cebal - Centro De Biotecnologia Agrícola E Agro-Alimentar Do Alentejo 7801-908 Beja (PT)**

(72) Inventors:
- **DA CONCEIÇÃO FERNANDES, Maria**
  **7800-492 BEJA (PT)**
- **ALVES FERREIRA CATURRA, Júnia Aparecida**
  **7800-721 SANTA CLARA DE LOUREDO (PT)**
- **ABREU CHORÃO DE QUELHAS DUARTE, Luís Jorge**
  **1500-183 LISBOA (PT)**

- **AZEVEDO MONIZ, Patrícia Maria**
  **1500-183 LISBOA (PT)**
- **FERREIRA MARQUES DA SILVA FERNANDES, TALITA**
  **12710-410 CRUZEIRO/ SÃO PAULO (BR)**
- **RUIVO TORRADO SANTANA, Ivone Maria**
  **7900-570 FERREIRA DO ALENTEJO (PT)**
- **DA SILVA GASPAR, Suelen**
  **37110-000 ELÓI MENDES, MINAS GERAIS (BR)**
- **RAPOSO SILVESTRE, Adriana Isabel**
  **7670-368 OURIQUE (PT)**
- **DE OLIVEIRA CARVALHEIRO ESTEVES AMARO, Florbela**
  **1750-126 LISBOA (PT)**
- **COSTA DO AMARAL RAMOS, António Marcos**
  **7800-072 BEJA (PT)**

(74) Representative: **Ferreira, Maria Silvina Clarke, Modet & Co Av. Casal Ribeiro, N°50-3° andar 1000-93 Lisboa (PT)**

(54) **PROCESS FOR THE PRODUCTION OF FORMIC ACID USING E. COLI STRAINS**

(57) The present patent application discloses a process where production of formic acid is made with the use of microorganism (*Escherichia coli*) that is capable to convert simple sugar into formic acid. Another asset is the improvement of formic acid production in the presence of furan compounds. The method comprises the use of mineral media/lignocellulosic hydrolysates with pentoses and/or hexoses as carbon source and is proved that formic acid is obtained. In the presence of 5-hydroxy-methyl furfural (HMF) an increment of 21-86 % is observed.

**EP 4 089 177 A1**

**Description**

**Technical field**

The present patent application relates to a process for the production of formic acid using *Escherichia coli* strains.

**Background art**

Formic acid is used primarily in dyeing, in the textile and leather industries; in rubber production; and as an intermediate in the chemical and pharmaceutical industries [1]. Lately formic acid has been used as a preservative and antibacterial agent in livestock feed. When sprayed on fresh hay or other silage, it arrests certain decay processes and causes the feed to retain its nutritive value longer, and so it is widely used to preserve winter feed for cattle.

The installed formic acid processes can be classified in four groups: 1) methyl formate hydrolysis, 2) preparation of free formic acid from formate, 3) hydrolysis of formamide, and 4) carbon dioxide capture. The first method was developed commercially by various companies into an economically feasible method [2][3]. Other chemical processes to obtain formic acid are also patented [4, 5]. Except for group 4) all these methods use raw material derived from fossil fuel. The fourth group has been recently created that is related with hydrogenation of carbon dioxide in alcohol [6] or the preparation of formic acid from carbon monoxide and water with tertiary amines [7]. In the search of alternatives resources for production of formic acid such as lignocellulosic biomass as a renewable resource it was proposed at least 2 patented methods that consist in the dehydration of carbohydrate followed by an hydrolysis step [8]. These processes are based on hydrolysis with mineral acid of material containing carbohydrates such as cellulose or starch[9] and also [10]. One of the disadvantages of these two last mentioned processes is that they use part of the lignocellulosic material, usually the liquid fraction obtained during the treatment with mineral acid, originating a solid that due to the applied process, has limited applications and only residual economic value.

**Summary**

The present patent application relates to a process for the production of formic acid using *Escherichia coli* strains in a liquid culture media comprising xylose and furan compounds.

Therefore, it is disclosed herein a process for the production of formic acid comprising the following steps:

- Preparation of a liquid mineral culture media comprising pentoses and/or hexoses as carbon source and at least one furan compound;
- Culturing of *E. coli* cells in the liquid media prepared in the previous step at 34 to 39 °C for 24 to 96h and at pH controlled 6.0 - 8.0.

In one embodiment, the at least one furan compound is 5-hydroxy-methyl furfural.

In one embodiment, the *E. coli* cells are from at least one strain selected from TG1 and Tuner.

In one embodiment, the culturing of *E.coli* is done in a stirred and microaerated bioreactor.

**Disclosure/Detailed Description**

The present patent application discloses a process where production of formic acid is made with the use of microorganism (*Escherichia coli*) that is capable to convert simple sugar into formic acid. Another asset is the improvement of formic acid production in the presence of furan compounds. The method comprises the use of mineral media/lignocellulosic hydrolysates with pentoses and/or hexoses as carbon source and is proved that formic acid is obtained. In the presence of 5-hydroxy-methyl furfural (HMF) an increment of 21-86 % is observed.

Thus, it is disclosed here for the first time a method for formic acid production using a current "biocatalyst" that is able to convert sugar from lignocellulosic material, being its productivity, surprisingly, increased by the presence of furans, that typically inhibit microbial metabolism.

One of the process to obtain formic acid is through the hydrolysis, with mineral acids, of material containing carbohydrate such as starch and lignocellulosic biomass. This is accomplished through the use of high concentration of acid and at temperature 90 to 110 °C that can jeopardize the reactor where the reaction take place. During this process, polymeric sugars (cellulose and hemicellulose) are solubilized in simple sugars and several potential metabolic inhibitors are derived, that can be grouped into three categories: 1) Furan derivatives (furfural and 5-hydroxymethylfurfural, from pentoses and hexose degradation, respectively), 2) Aliphatic acids (acetic acid is a common structural constituent of the hemicellulosic fraction and formic and levulinic acids are derived from sugar degradations reactions) and 3) Lignin derived phenolic compounds. To produce formic acid with high yields, the acid hydrolysis should be performed with severe conditions in order to convert the simple sugar resultant from the polymers into formic acid.

Conversely, the applied temperature can be increased with the reduction of mineral acid concentration being the resultant process gentler for equipment. Although, since hemicellulose is fragmented originating a liquid fraction rich in xylose and also contains some the inhibitors mentioned above that may include formic acid in lower concentration. The present application shows a microorganism capable of using xylose/glucose as sugar source in the fermentation with the capacity to produce formic acid, beside other fermentation products such as ethanol and acetic acid. The microorganism of the present application is the bacteria *Escherichia coli,* that usually are used in molecular biology experiments, uses xylose as sugar source and produce formic acid and other fermentation products. When furan compound, are added to mineral media, the *E.coli* strains presented an increment of 21-86 % of formic acid production. Since *E. coli* strains are capable to use glucose as carbon source during fermentation process (Gonzalez et al 2017) [4], this same process can be applied to use the glucose coming from the resultant solid obtained during acid treatment of lignocellulosic material.

The present application shows a microorganism capable of using xylose/glucose as sugar source in the fermentation with the capacity to produce formic acid, beside other fermentation products such as ethanol and acetic acid. The microorganism of the present application is the bacteria *Escherichia coli* that usually are used in molecular biology experiments. *E. coli* used xylose as sugar source and produce formic acid and other fermentation products.

A) Formulation of chemically defined liquid media, called MMS, was based on media M9[11], AM1 [13], NBS [13], YNB, [14] and MB [15] whose composition is shown in Table 1, that simulates a lignocellulosic hydrolysate and that was specifically formulated for this work. The solutions of vitamins, trace elements, inhibitors and betaine were previously sterilized using membrane filters of 0.22 $\mu$m (Gelman Sciences, Ann Arbor, Michigan, EUA) and the remaining solutions of the media were sterilized in an autoclave (121°C, 15 minutes). 2.0 mL/L of trace element solution and 1.0 mL/L of vitamin solution were used in the media MMS. The preparation of the media in the present application was made in a flow chamber.

**Table** 1 Composition of the MMS media

| | Component | Formula | Mmol/L |
|---|---|---|---|
| | Xylose | $C_5H_{10}O_5$ | 133.21 |
| | Diammonium hydrogen phosphate | $(NH_4)_2HPO_4$ | 19.91 |
| | Ammonium dihydrogen phosphate | $NH_4H_2PO_4$ | 7.56 |
| | Potassium dihydrogen phosphate | $KH_2PO_4$ | 28.29 |
| | Dipotassium hydrogen phosphate | $K_2HPO_4$ | 71.70 |
| | Magnesium sulfate heptahydrate | $MgSO_4.7H_2O$ | 1.19 |
| | Potassium hydroxide | KOH | 0.99 |
| Vitamin solution | Betaine | $C_5H_{10}NO_2$ | 1.32 |
| | Biotin | $C_{10}H_{16}N_2O_3S$ | 0.004 |
| | Thiamine | $C_{12}H_{17}N_4OS+$ | 0.019 |
| | Proline | $C_5H_9NO_2$ | 1.99 |
| Trace element solution | Ferric Chloride Hexahydrate | $FeCl3.6H_2O$ | 0.004 |
| | Cobalt chloride hexahydrate | $CoCl_2.6H_2O$ | 0.0007 |
| | *Copper chloride dihydrate* | $CuCl_2.2H_2O$ | 0.0001 |
| | Zinc chloride | $ZnCl_2.4H_2O$ | 0.002 |
| | Sodium molybdate dihydrate | $Na_2MoO4$ | 0.0004 |
| | Boric acid | $H_3BO_3$ | 0.001 |
| | Manganese chloride tetrahydrate | $MnCl_2.4H_2O$ | 0.22 |

B) The growth of *E. coli* strains in Luria Bertani media and MMS media were performed in baffled 1L Erlenmeyers starting with an initial optical density (OD) at 600nm o, 0.2. The experiment was carried in orbital (TEQ/OSFT-LS, Portugal) shaker 200 rpm at 37°C for 24h.

Preparation of inocula: A colony was collected from Luria-Bertani solid medium with 2% xylose and suspended in Luria-Bertani liquid medium with 2% xylose. In case of preparation of inocula for MMS the strains were obtained from cryovials at - 80°C in the presence of glycerol (20%), previously grown in LB media. Assay were performed in 1L baffled Erlenmeyers 200 rpm at 37°C and the cell were recovered at the end of the exponential phase. After this time, the strains were stored in cryovials at - 80°C in the presence of glycerol (20%). Strains preserved in glycerol were used as stock for the subsequent assays.

C) The strains of *E. coli* prepared as inocula, collected during exponential phase when growing in MMS media, were thawed and centrifuged at 12000 rpm. Pellet was resuspended in MMS, reaching an initial OD (600 nm) of approximately 0.2. The fermentation was conducted in mini reactors, with a final volume of 200 mL. Component of media were added top each reactor and different concentrations of inhibitors (furan compounds) . When necessary, KOH 2N was added, with agitation, to each reactor for pH adjustment to 7.0. The volume of each mini reactor was completed with sterile distilled water to attain 200 mL. During fermentation assay pH was controlled at 6.0 to 8.0, preferably at 6.9 to 7.1 by the automatic addition of base (KOH, 2N).

The entire experiment was conducted at least in duplicate, under agitation of 250 rpm at 37°C, during 72 hours. Samples of 2 mL were periodically and aseptically removed with the aid of sterile syringes attached to the mini reactors in the times T0, T3, T6, T9, T24, T32, T48, T56 and T72, pH and consumed base volume was monitored. When each sample was taken, the cell concentration was immediately measured by a double beam spectrophotometer (Helios Alfa, Thermo Scientific), afterwards were centrifuged at 12,000 rpm for 5 minutes at 4°C and the supernatant stored for later analysis. Data of optical density, sugars consumption and fermentation products formation were measured by High performance liquid chromatography - HPLC analysis, were used for stoichiometric calculations.

D) After thawing supernatants were filtered through a 0.20 $\mu$m porosity ultrafiltration membrane, analyzed by HPLC and processed according to [16]. Briefly, sugars (monosaccharides) and organic acids were analyzed by HPLC (Dionex Ultimate 3000 system (Thermo Scientific, USA), equipped with a Diode Array detector DAD-3000(Thermo Scientific, USA. An Aminex HPX-87H (7.8 × 300 mm) cation exchange column with a micro-guard cation H cartridges both from Bio-Rad (USA, Hercules, CA), were used at 50°C with 0.01 N $H_2SO_4$ 5 mM aqueous solution as mobile phase (since it allows to simultaneously measure the sugars and organic acids), with the flow of 0.6 mL min$^{-1}$. Detection was performed using the refractive index detector. Concentrations were determined with an external standard, through standard curves for each analyzed component.

## Examples

### Example 1- Preparation of chemical defined media MMS

For the preparation of chemically defined liquid media, called MMS it was necessary to prepare the following stock solutions: xylose (200g/L), di-ammonium hydrogen phosphate 26,3 g/L, ammonium dihydrogen phosphate 8.7 g/L, 1M Potassium Phosphate Buffer - pH 7.2, magnesium sulphate heptahydrate 36.96 g/L, Calcium chloride dihydrate 4.41 g / L, vitamin solution, proline 23 g/L, potassium hydroxide 2 and trace elements solution.

Vitamin solution: 3.840 g of betaine, 0.025 g of biotin and 0.127 g of thiamine were transferred to a 25 mL volumetric flask with the aid of a nozzle containing distilled water, adjusting the volume through the meniscus. After the complete dissolution of the vitamins, the solution was sterilized by filtration (0.22 $\mu$m pore filter). The solution was stored at 4°C and protected from light (with aluminum foil).

Trace elements solution in HCl 120 mM is composed by the following components

| Reagent | [g/L] |
|---|---|
| $FeCl_3.6H_2O$ | 0.24 |
| $CoCl_2.6H_2O$ | 0.03 |
| $CuCl_2.2H_2O$ | 0.015 |
| $ZnCl_2$ | 0.03 |
| $Na_2MoO_4.2H_2O$ | 0.03 |
| $H_3BO_3$ | 0.0075 |
| $MnCl_2.4H_2O$ | 0.05 |

The masses of the trace elements in the table above were transferred to a 200 mL volumetric flask with the aid of a nozzle containing distilled water. In the hood, 2.0 mL of 37% HCl was added to the flask and was made up to volume with distilled water. After the complete dissolution of the trace elements, the solution was distributed in Schott flasks and sterilized at 121°C for 20 min. in autoclave. The solution was stored at 4°C and protected from light (with aluminum foil).

The culture medium was prepared inside the laminar flow chamber and all the material used in its preparation was previously sterilized at 121°C for 20 min, as well as the distilled water used to complete the volume of the medium. All inhibitor/ furan compounds solutions were sterilized by filtration.

Below, the quantities of each solution necessary to prepare 1000 mL of medium are followed, the volume of inhibitor added is subtracted from the volume of sterile distilled water:

| Stock solution | Concentration | Volume (mL) |
|---|---|---|
| Xylose | 200 g/L [10x] | 100 |
| di-ammonium hydrogen phosphate | 26,3 g/L [10x] | 100 |
| ammonium dihydrogen phosphate | 8,7 g/L[10x] | 100 |
| Potassium Phosphate Buffer - pH 7.2 | 1M [10x] | 100 |
| magnesium sulphate heptahydrate | 36,96 g/L [100x] | 10 |
| Calcium chloride dihydrate | 4,41 g/L [100x] | 10 |
| Vitamins solution | [1000x] | 1 |
| Proline | 23 g/L [100x] | 10 |
| Trace elements | [500x] | 2 |
| Sodium hydroxyde | 5,6 g/L [100x] | 10 |
| Steril destiled water | | $557 - V_{inhibitor}$ |

**Example 2 Suitability of MMS for *E.coli* growth**

The following experiment shows results obtained with *E .coli* strains Tuner and TG1 for comparison of adequacy of formulated MMS (Table 1) in comparison to Luria Bertani (LB) media both containing 2% xylose as sugar source.

The growth of *E. coli* strains TG1 and Tuner were performed in Luria Bertani (LB) media and MMS media in baffled 1L Erlenmeyers starting with an initial optical density (OD) at 600nm of 0.2. The experiment was carried in orbital (TEQ/OSFT-LS, Portugal) shaker 200 rpm at 37°C for 24h form MMS experiments and 7h for LB media.

Preparation of inocula: A colony was collected from LB solid medium with 2% xylose and suspended in LB liquid medium with 2% xylose. In case of preparation of inocula for MMS the strains were obtained from cryovials at - 80°C in the presence of glycerol (20%), previously grown in LB media. Assay were performed in 1L baffled Erlenmeyers 200 rpm at 37°C and the cell were recovered at the end of the exponential phase. After this time, the strains were stored in cryovials at - 80°C in the presence of glycerol (20%). Strains preserved in glycerol were used as stock for the subsequent assays.

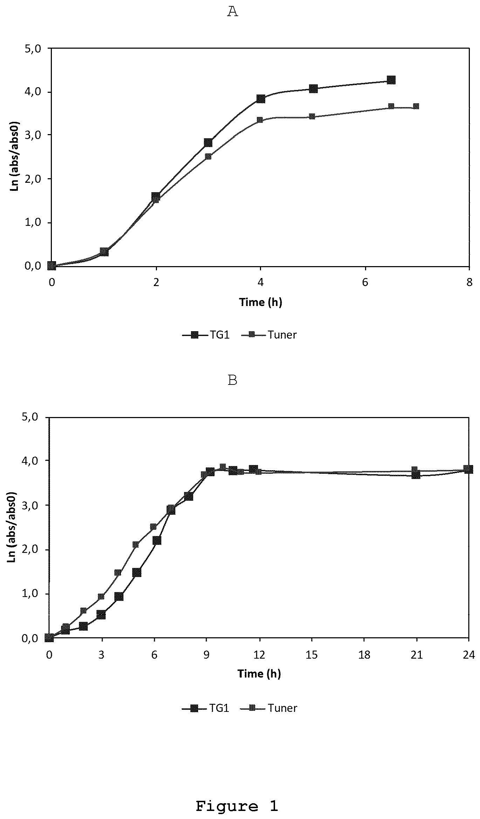

The results are shown in Figure 1 A and B, shows the kinetic growth for *E.coli* TG1 and Tuner. It was possible to calculate the latency phase, the specific speed of growth with the curves obtained in Fig 1 that are represented in Table 2.

**Table 2.** Latency phase ($\lambda$ specific growth rate ($\mu$ of the strains tested in 1L baffled Erlenmeyer

| Strain-media | $\lambda$ (h) | $\mu.h^{-1}$ |
|---|---|---|
| TG1 MMS | 2.08 | 0.55 |
| Tuner MMS | 0.63 | 0.45 |
| TG1 LB | 0,00 | 0.87 |
| Tuner LB | 0,00 | 0.72 |

Tuner and TG1 showed a growth curve similar to that obtained in LB medium. The latency phase obtained in experiments with MMS media, 0.63 and 2.08h, is due to adaptation of the strains to this media, since the inocula was prepared from a stock grown in LB media. The specific rate of growth was slightly lower in experiments with MMS media, which can be justified by the poor media composition in comparison to LB media composed by yeast extract(5g/L), tryptone (10 g/L)and sodium chloride (5 g/L).

**Example 3 Effect of furan compounds in the production of formic acid in MMS**

The strains of *E. coli* TG1 and Tuner prepared as inocula were collected during exponential phase when growing in MMS media in a baffled Erlenmeyer. After being thawed and centrifuged at 12000 rpm, pellet was resuspended in MMS, reaching an initial OD (600 nm) of approximately 0.2. The fermentation was conducted in mini reactors, with a final volume of 200 mL. Component of media were added top each reactor and different concentrations of inhibitors, according to Table 3. When necessary, KOH 2N was added, with agitation, to each reactor for pH adjustment to 7.0. The volume of each mini reactor was completed with sterile distilled water to attain 200 mL. During fermentation assay pH was controlled by the automatic addition of base (KOH, 2N) .

**Table 3-** Kinetic and stoichiometric parameters of fermentations with *E. coli* strain TG1 and Tuner. Qp was calculated at 72h.

| Strain | Furan | [g/L] | $Q_{xyl}$ (g/L h) | Produced (g/L) FA | $Q_P$ (g FA/Lh) | $Y_{PS}$ (g/g) | $Q_{PF}/Q_{PC}$[a] |
|--------|-------|-------|------|------|------|------|------|
| TG1 | Control | - | 0,20 | 4,93 | 0,068 | 0,34 | 1,00 |
| | F | 0.6 | 0,14 | 0,52 | 0,007 | 0,05 | 0,10 |
| | F | 1.2 | 0,14 | 0,00 | 0,000 | 0,00 | 0,00 |
| | F | 2.4 | 0,03 | 0,00 | 0,000 | 0,00 | 0,00 |
| | HMF | 0.5 | 0,28 | 5,96 | 0,083 | 0,30 | 1,21 |
| | HMF | 1 | 0,27 | 7,27 | 0,101 | 0,38 | 1,47 |
| | HMF | 2 | 0,26 | 6,90 | 0,096 | 0,37 | 1,40 |
| Tuner | Control | - | 0,26 | 3,70 | 0,051 | 0,20 | 1,00 |
| | F | 0.6 | 0,23 | 4,20 | 0,058 | 1.12 | 1,14 |
| | F | 1.2 | 0,08 | 1,86 | 0,026 | 0.99 | 0,50 |
| | F | 2.4 | 0,00 | 0,00 | 0,000 | 1.14 | 0,00 |
| | HMF | 0.5 | 0,28 | 5,96 | 0,083 | 1.06 | 1,61 |
| | HMF | 1 | 0,27 | 6,11 | 0,085 | 0.84 | 1,65 |
| | HMF | 2 | 0,26 | 6,90 | 0,096 | 0.87 | 1,86 |

a- $Q_{PF}/Q_{PCa}$ correspond to the ratio between formic acid productivity in the presence of furan compound and the formic productivity in the control

The entire experiment was conducted at least in duplicate, under agitation of 250 rpm at 37°C, during 72 hours. Samples of 2 mL were periodically and aseptically removed with the aid of sterile syringes attached to the mini reactors in the times T0, T3, T6, T9, T24, T32, T48, T56 and T72, pH and consumed base volume was monitored. When each sample was taken, the cell concentration was immediately measured by a double beam spectrophotometer (Helios Alfa, Thermo Scientific), afterwards were centrifuged at 12,000 rpm for 5 minutes at 4°C and the supernatant stored for later analysis. Data of optical density, sugars consumption and fermentation products formation were measured by High performance liquid chromatography - HPLC analysis, were used for stoichiometric calculations

Samples collected along the fermentation were frozen and after thawed were filtered for HPLC analysis and processed according to [16]. Briefly, sugars (monosaccharides) and organic acids were analyzed by HPLC (Dionex Ultimate 3000 system (Thermo Scientific, USA), equipped with a Diode Array detector DAD-3000(Thermo Scientific, USA. An Aminex HPX-87H (7.8 × 300 mm) cation exchange column with a micro-guard cation H cartridges both from Bio-Rad (USA, Hercules, CA), were used at 50°C with 0.01 N $H_2SO_4$ 5 mM aqueous solution as mobile phase (since it allows to simultaneously measure the sugars and organic acids), with the flow of 0.6 mL/min. Detection was performed using the refractive index detector. Concentrations were determined with an external standard, through standard curves for each analyzed component

*Calculations*

The evaluation of growth was monitored by measuring optical density (OD) or absorbance (Abs) at 600 nm ,on the samples taken over time. The kinetic was obtained by graphing the ln of the ratio of Abs measured in time (Abst)/Abs measured in time 0 (Abs0) as a function of time (h).

The specific growth rate ($\mu$, h$^{-1}$) was calculated to compare the growth in different concentrations of inhibitors and obtained from the slope measured during the exponential phase. The latency phase ($\lambda$, h) was obtained by dividing the ordinate at the origin by the slope of the equation obtained in the specific growth rate. The xylose consumption rate ($Q_{xyl}$) was obtained using the equation 1.

$$Q_{xyl} = \frac{Cxyl_t - Cxyl_0}{t} \qquad (1)$$

Where *Cxyl$_t$* corresponds to the measured xylose concentration (g/L) at time t(h) and *Cxyl$_0$* corresponds to the xylose concentration (g/L) at time 0h; and t corresponds to the time measured in t(h).

To determine the products yield (Y$_{p/s}$) of formic acid of the fermentation, equations (3) was used, taking into account the whole fermentation elapsed time for the experiment.

$$Y\,^{p}/_{S} = \frac{\text{grams of generated product}}{\text{grams } of \text{ consumed substrate}} = \frac{Pf-Po}{So-Sf} \qquad (3)$$

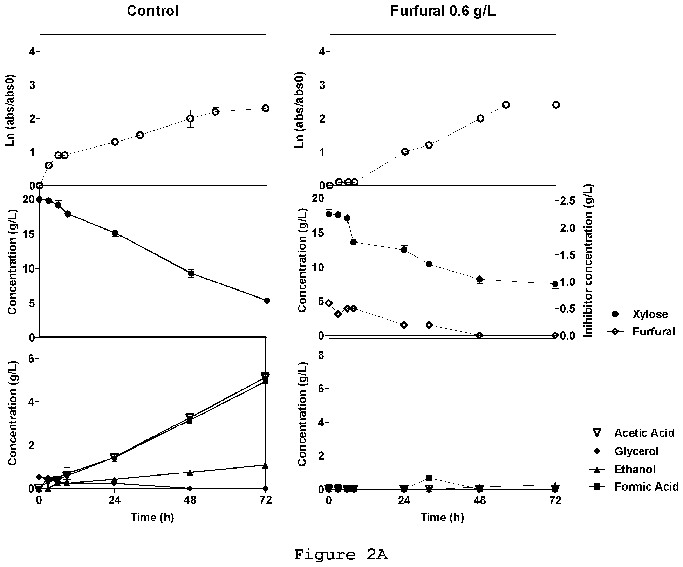

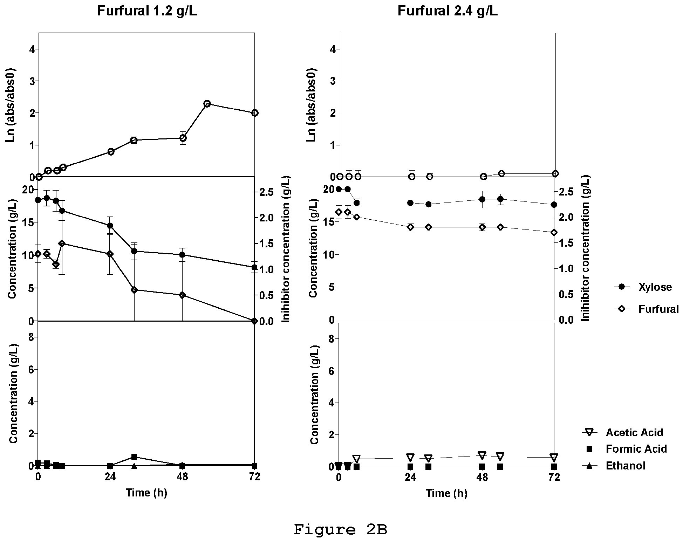

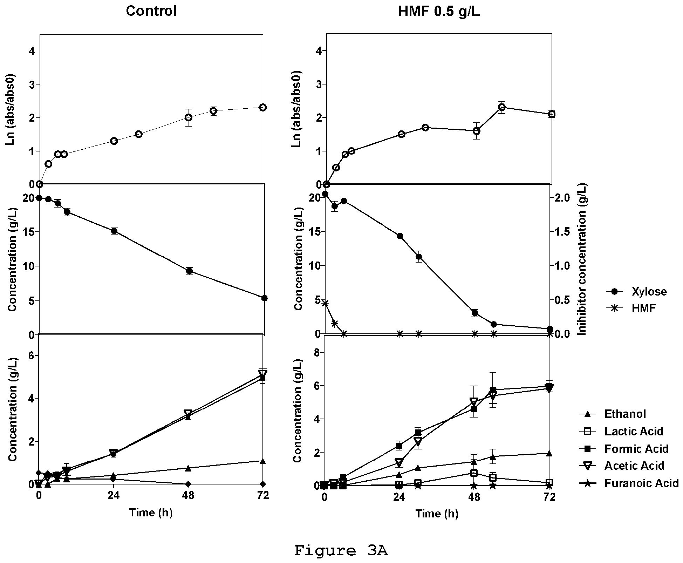

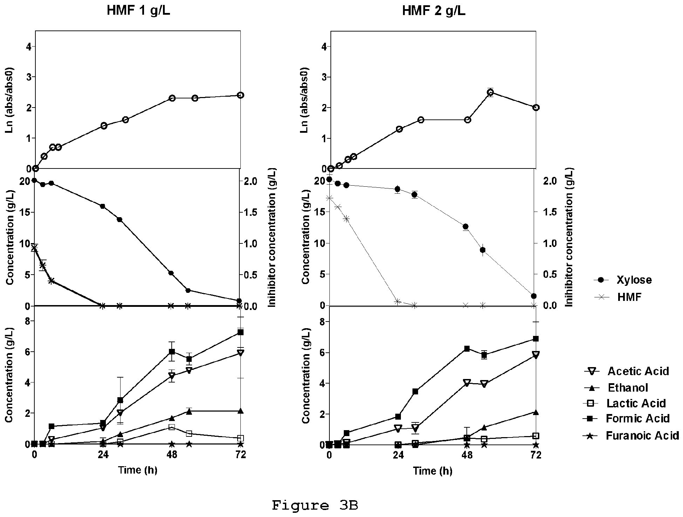

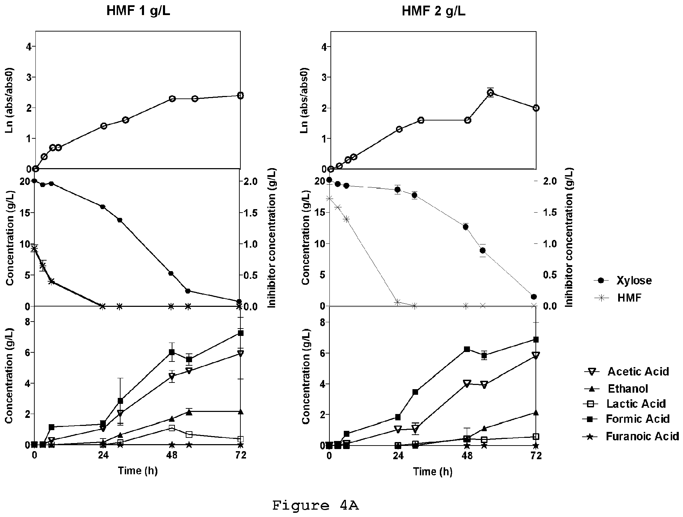

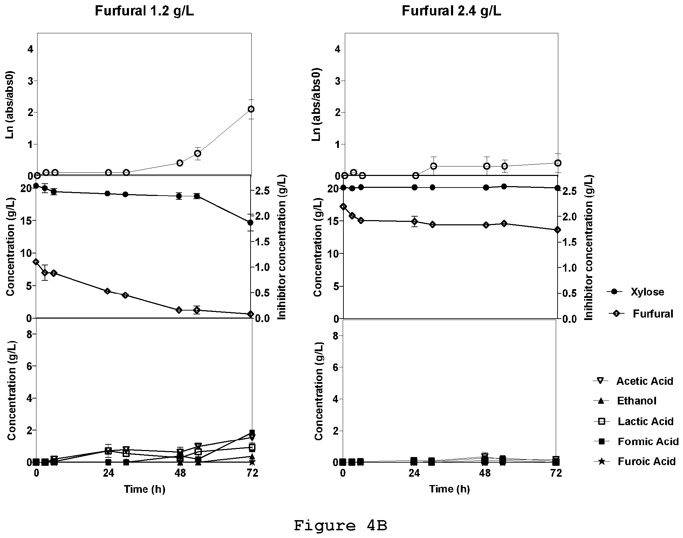

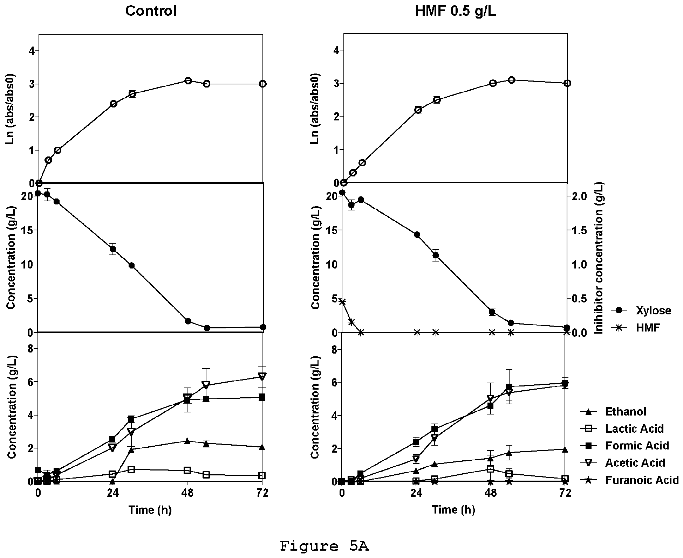

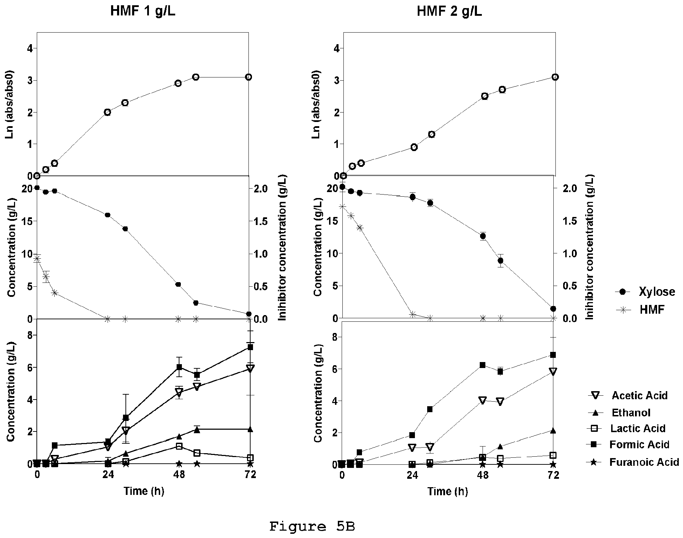

Graphical representation of the fermentation process for TG1 and Tuner in the presence of two furan compound: Furfural and 5-hydroxymethylfurfural and shown in Figures 2-5. Kinetic and stoichiometric parameters of fermentations with *E. coli* strain TG1 and Tuner are indicated in Table 3. The observation of Fig 2-5 is possible to see that formic acid is produced in higher quantity in the presence of 5-hydroxymethylfurfural, for both strain TG1 and Tuner. Conversely, furfural has a negative impact on growth and in formic acid production. The ratio obtained for the productivity of formic acid in the presence of 5-hydroxymethylfurfural is higher than the control assay. The increment of formic acid production varies from 21 to 86%. (Table 3).

**Brief description of drawings**

For easier understanding of this application, figures are attached in the annex that represent the preferred forms of implementation which nevertheless are not intended to limit the technique disclosed herein.

Figure 1 shows the growth of *E.coli* strains TG1 and Tuner with LB media (A) and MMS (B) 2% xylose in baffled Erlenmeyer

Figures 2A and 2B show the fermentation profile of *E. coli* TG1 in MMS media in the absence (control) and in the presence of Furfural inhibitor at concentrations of 0.6, 1.2 and 2.4 g/L: growth kinetics, xylose consumption and formation of products during 72 hours of fermentation process.

Figures 3A and 3B show the fermentation profile of *E. coli* TG1 in MMS media in the absence (control) and in the presence of 5-Hydroxymethylfurfural inhibitor at concentrations of 0.5, 1.0 and 2.0 g/L: growth kinetics, xylose consumption and formation of products during 72 hours of fermentation process.

Figures 4A and 4B show the fermentation profile of E. coli Tuner in MMS media in the absence (control) and in the presence of 5-Hydroxymethylfurfural inhibitor at concentrations of 0.5, 1.0 and 2.0 g/L: growth kinetics, xylose consumption and formation of products during 72 hours of fermentation process.

Figures 5A and 5B show the fermentation profile of *E. coli* Tuner in MMS media in the absence (control) and in the presence of Furfural inhibitor at concentrations of 0.6, 1.2 and 2.4 g/L: growth kinetics, xylose consumption and formation of products during 72 hours of fermentation process.

**Description of the embodiments**

Now, preferred embodiments of the present application will be described in detail. However, these are not intended to limit the scope of this application.
The present patent application discloses a process for the production of formic acid comprising the following steps:

- Preparation of a liquid mineral culture media comprising pentoses and/or hexoses as Carbon source and at least one furan compound; Culturing of *E. coli* cells in the liquid media prepared in the previous step at 34 to 39 °C for 24 to 72h.

In one embodiment, the at least one furan compound is 5-hydroxy-methyl furfural.
In one embodiment, the *E. coli* cells are from at least one strain selected from TG1 and Tuner.
In one embodiment, the culturing of *E.coli* is done in 1L baffled Erlenmeyers at 200 rpm.
This description is of course not in any way restricted to the forms of implementation presented herein and any person with an average knowledge of the area can provide many possibilities for modification thereof without departing from the general idea as defined by the claims. The preferred forms of implementation described above can obviously be combined with each other. The following claims further define the preferred forms of implementation.

**References**

1. Reutemann W, Kieczka H (2005) Acid Formic. In: Ullmann's Encyclopedia of Industrial Chemistry, 5th Editio. Wiley-VCHVerlagGmbH&Co.KGaA, Weinheim, pp 1-22

2. SCHAUB T, MARIA FD, ROCCO P, et al (2013) WO2013004577 (A1)-PROCESS FOR THE PREPARATION OF FORMIC ACID BY REACTING CARBON DIOXIDE WITH HYDROGEN

3. Hietala J, Vouri A, Johnsson P, et al (2016) Formic Acid. In: Ullmann's Encyclopedia of Industrial Chemistry. Wiley-VCHVerlagGmbH&Co.KGaA, Weinheim, pp 1-22

4. Gonzalez JE, Long CP, Antoniewicz MR (2017) Comprehensive analysis of glucose and xylose metabolism in Escherichia coli under aerobic and anaerobic conditions by 13C metabolic flux analysis. Metab Eng 39:9-18. https://doi.org/10.1016/j.ymben.2016.11.003

5. RICCI A, SBRANA G, BRACA G (1987) EP0248259 (A1) - Process for producing formic acid by carbonylation of hydroxyalkylamines and hydrolysis of the hydroxyalkylformamides produced

6. KENT AG (1985) EP151510 (A1)-Production of formate salts

7. BROCKHAUS RD+ DE3903664 (A1) - Process for the preparation of formic acid from carbon monoxide and water. 1980

8. Palmqvist E (2000) Fermentation of lignocellulosic hydrolysates. II: inhibitors and mechanisms of inhibition. Bioresour Technol 74:25-33. https://doi.org/10.1016/S0960-8524(99)00161-3

9. STEPHEN WF (2019) BRPI1009266 (A2) - PRODUCTION OF FORMIC ACID

10. MULLEN BD;, YONTZ DJ, LEIBIG CM (2017) US2015291499 (A1)-PROCESS TO PREPARE LEVULINIC ACID

11. Elbing K, Brent R (2002) Media Preparation and Bacteriological Tools. In: Current Protocols in Molecular Biology. pp 1.1.1-1.1.7

12. Martinez A, Grabar TB, Shanmugam KT, et al (2007) Low salt medium for lactate and ethanol production by recombinant Escherichia coli B. Biotechnol Lett 29:397-404. https://doi.org/10.1007/s10529-006-9252-y

13. Martinez A, Grabar TB, Shanmugam KT, et al (2007) Low salt medium for lactate and ethanol production by recombinant Escherichia coli B. Biotechnol Lett 107:397-404. https://doi.org/10.1007/s10529-006-9252-y

14. Shadomy S, Espinel Ingroff A (1980) Susceptibility Testing with Antifungal Drugs,. In: Lennete E, Balos A, Hausler Jr. W, Truant J (eds) Manual Clinical Microbiology, U.S. Dept. American Society for Microbiology, Washington, D.C., pp 647-653

15. Veríssimo A, Morais P MICROBIOLOGIA- Medição do crescimento de E. coli.

16. Fernandes MC, Ferro MD, Paulino AFC, et al (2018) Comparative study on hydrolysis and bioethanol production from cardoon and rockrose pretreated by dilute acid hydrolysis. Ind Crops Prod 111:633-641. https://doi.org/10.1016/j.indcrop.2017.11.037

**Claims**

1. Process for the production of formic acid comprising the following steps:

   - Preparation of a liquid mineral culture media comprising xylose as sugar source and at least one furan compound;
   - Culturing of *E. coli* cells in the liquid media prepared in the previous step at 34 to 39 °C for 24 to 72h.

2. Process for the production of formic acid according to claim 1, wherein the at least one furan compound is 5-hydroxymethyl furfural.

3. Process for the production of formic acid according to claim 1, wherein the *E. coli* cells are from at least one strain selected from TG1 and Tuner.

4. Process for the production of formic acid according to claim 1, wherein the culturing of *E.coli* is carried out in an stirred and aerated bioreactor.

A

B

Figure 1

Figure 2A

Figure 2B

Figure 3A

Figure 3B

HMF 1 g/L    HMF 2 g/L

Figure 4A

**Figure 4B**

Figure 5A

Figure 5B

Europäisches
Patentamt

European
Patent Office

Office européen
des brevets

# EUROPEAN SEARCH REPORT

Application Number

EP 21 17 4190

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LOPES MATEUS SCHREINER GARCEZ ET AL: "Polyhydroxyalkanoate biosynthesis and simultaneous remotion of organic inhibitors from sugarcane bagasse hydrolysate bysp", JOURNAL OF INDUSTRIAL MICROBIOLOGY & BIOTECHNOLOGY, BASINGSTOKE, GB, vol. 41, no. 9, 25 July 2014 (2014-07-25), pages 1353-1363, XP037121855, ISSN: 1367-5435, DOI: 10.1007/S10295-014-1485-5 [retrieved on 2014-07-25] * page 1355 - page 1356; table 6 * | 1-4 | INV. C12P7/40 C12P7/54 C12P7/56 C12P7/06 C12P7/10 |
| A | CESARIO M. TERESA ET AL: "Enhanced bioproduction of poly-3-hydroxybutyrate from wheat straw lignocellulosic hydrolysates", NEW BIOTECHNOLOGY, vol. 31, no. 1, 1 January 2014 (2014-01-01), pages 104-113, XP055853691, NL ISSN: 1871-6784, DOI: 10.1016/j.nbt.2013.10.004 * page 105 - page 107; figures 1,2 * | 1-4 | |
| A | EVA PALMQVIST ET AL: "Fermentation of lignocellulosic hydrolysates. II: inhibitors and mechanisms of inhibition", BIORESOURCE TECHNOLOGY, vol. 74, no. 1, 1 August 2000 (2000-08-01), pages 25-33, XP055105940, ISSN: 0960-8524, DOI: 10.1016/S0960-8524(99)00161-3 * page 26 - page 27; figure 1 * | 1-4 | |

TECHNICAL FIELDS
SEARCHED (IPC)

C12P

-/--

The present search report has been drawn up for all claims

| Place of search | Date of completion of the search | Examiner |
|---|---|---|
| Berlin | 8 November 2021 | Schneider, Patrick |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 21 17 4190

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 8 138 371 B2 (FITZPATRICK STEPHEN W [US]; BIOFINE TECHNOLOGIES LLC [US]) 20 March 2012 (2012-03-20) * column 3 - column 5 * ----- | 1-4 | |

TECHNICAL FIELDS
SEARCHED     (IPC)

The present search report has been drawn up for all claims

| Place of search | Date of completion of the search | Examiner |
|---|---|---|
| Berlin | 8 November 2021 | Schneider, Patrick |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 21 17 4190

This annex lists the patent family members relating to the patent documents cited in the above-mentioned European search report.
The members are as contained in the European Patent Office EDP file on
The European Patent Office is in no way liable for these particulars which are merely given for the purpose of information.

08-11-2021

| Patent document cited in search report | | Publication date | Patent family member(s) | | Publication date |
|---|---|---|---|---|---|
| US 8138371 | B2 | 20-03-2012 | BR | PI1009266 A2 | 27-08-2019 |
| | | | CA | 2792849 A1 | 16-09-2010 |
| | | | CN | 102414158 A | 11-04-2012 |
| | | | EP | 2406209 A1 | 18-01-2012 |
| | | | HK | 1169374 A1 | 25-01-2013 |
| | | | US | 2010234638 A1 | 16-09-2010 |
| | | | US | 2012172625 A1 | 05-07-2012 |
| | | | US | 2014323759 A1 | 30-10-2014 |
| | | | WO | 2010104722 A1 | 16-09-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2013004577 A1, SCHAUB T, MARIA FD, ROCCO P
- EP 0248259 A1, RICCI A, SBRANA G, BRACA G
- EP 151510 A1, KENT AG
- DE 3903664 A1
- US 2015291499 A1, MULLEN BD;, YONTZ DJ, LEIBIG CM

### Non-patent literature cited in the description

- Acid Formic. **REUTEMANN W ; KIECZKA H.** Ullmann's Encyclopedia of Industrial Chemistry. Wiley-VCHVerlagGmbH&Co.KGaA, 2005, 1-22
- Formic Acid. **HIETALA J ; VOURI A ; JOHNSSON P et al.** Ullmann's Encyclopedia of Industrial Chemistry. Wiley-VCHVerlagGmbH&Co.KGaA, 2016, 1-22
- **GONZALEZ JE ; LONG CP ; ANTONIEWICZ MR.** Comprehensive analysis of glucose and xylose metabolism in Escherichia coli under aerobic and anaerobic conditions by 13C metabolic flux analysis. *Metab Eng,* 2017, vol. 39, 9-18, https://doi.org/10.1016/j.ymben.2016.11.003
- **PALMQVIST E.** Fermentation of lignocellulosic hydrolysates. II: inhibitors and mechanisms of inhibition. *Bioresour Technol,* 2000, vol. 74, 25-33, https://doi.org/10.1016/S0960-8524(99)00161-3
- **STEPHEN WF.** *BRPI1009266 (A2) - PRODUCTION OF FORMIC ACID,* 2019
- **ELBING K ; BRENT R.** Media Preparation and Bacteriological Tools. *Current Protocols in Molecular Biology,* 2002, 1.1.1-1.1.7
- **MARTINEZ A ; GRABAR TB ; SHANMUGAM KT et al.** Low salt medium for lactate and ethanol production by recombinant Escherichia coli B. *Biotechnol Lett,* 2007, vol. 29, 397-404, https://doi.org/10.1007/s10529-006-9252-y
- **MARTINEZ A ; GRABAR TB ; SHANMUGAM KT et al.** Low salt medium for lactate and ethanol production by recombinant Escherichia coli B. *Biotechnol Lett,* 2007, vol. 107, 397-404, https://doi.org/10.1007/s10529-006-9252-y
- Susceptibility Testing with Antifungal Drugs. **SHADOMY S ; ESPINEL INGROFF A.** Manual Clinical Microbiology. U.S. Dept. American Society for Microbiology, 1980, 647-653
- **VERÍSSIMO A ; MORAIS P.** *MICROBIOLOGIA-Medição do crescimento de E. coli.*
- **FERNANDES MC ; FERRO MD ; PAULINO AFC et al.** Comparative study on hydrolysis and bioethanol production from cardoon and rockrose pretreated by dilute acid hydrolysis. *Ind Crops Prod,* 2018, vol. 111, 633-641, https://doi.org/10.1016/j.indcrop.2017.11.037